# EUROPEAN PATENT SPECIFICATION

(11) **EP 2 315 750 B1**
(45) Date of publication and mention of the grant of the patent: **07.11.2012**
(21) Application number: 09774029.4
(22) Date of filing: 15.06.2009
(51) Int. Cl.: C07D 213/08, C07D 213/12, C07D 213/26, C07D 213/32

(54) **IMPROVED PROCESS FOR THE PREPARATION OF 2-TRIFLUOROMETHYL-5-(1-SUBSTITUTED)ALKYLPYRIDINES**
VERBESSERTES VERFAHREN ZUR HERSTELLUNG VON 2-TRIFLUORMETHYL-5-(1-SUBSTITUIERTEN) ALKYLPYRIDINEN
PROCÉDÉ PERFECTIONNÉ POUR LA FABRICATION DE 2-TRIFLUOROMÉTHYL-5-(1-SUBSTITUANT)ALKYLPYRIDINES

(30) Priority: 01.07.2008 US 77189
(43) Date of publication of application: 04.05.2011
(73) Proprietor: Dow AgroSciences LLC, Indianapolis IN 46268-1054 (US)
(72) Inventor: BLAND, Douglas, Midland MI 48642 (US); ROTH, Gary, Midland MI 48640 (US)
(74) Representative: Weiss, Wolfgang
(86) International application number: PCT/US2009/047387
(87) International publication number: WO 2010/002577

(56) References cited:
- EP-A- 1 340 747
- WO-A-2008/018917
- WO-A-2008/066558
- WO-A-2008/097234
- US-A1- 2007 203 191
- US-A1- 2008 108 667
- US-A1- 2009 029 863
- COOKE J W ET AL: "Efficient synthesis of 2-trihalomethyl-5-cyanopyridines" TETRAHEDRON LETTERS, ELSEVIER, AMSTERDAM, vol. 39, no. 43, 22 October 1998 (1998-10-22), pages 7965-7966, XP004137854 ISSN: 0040-4039

## Description

### BACKGROUND OF THE INVENTION

This application claims the benefit of United States Provisional Application Serial Number 61/077,189 filed on July 1, 2008.

The present invention concerns an improved process for the preparation of 2-tri tluoromethyl-5-(1-substituted)alkylpyridines.

2-Trifluoromethyl-5-(1-alkylthio)alkylpyridines are useful intermediates for the preparation of certain new insecticides; see, for example, U.S. Patent Publications 2005/0228027 and 2007/0203191. 4-Alkoxy-1,1,1-trifluoro-3-buten-2-ones are useful intermediates for preparing 2-trifluoromethyl-5-(1-alkylthio)alkylpyridines; see, for example, U.S. Patent Publication 2008/0033180 A1. Unfortunately, 4-alkoxy-1,1,1-trifluoro-3-buten-2-ones are relatively expensive and somewhat unstable, i.e., it is recommended that they be stored under refrigeration. It would be desirable to have a process for preparing 2-trifluoromethyl-5-(1-substituted)alkylpyridines in general and 2-trifluoromethyl-5-(1-alkylthio)alkylpyridines in particular in which the 4-alkoxy-1,1,1-trifluoro-3-buten-2-ones could be avoided, thus eliminating the transportation and storage issues associated with these raw materials.

### SUMMARY OF THE INVENTION

The present invention concerns an improved process for the preparation of 2-trifluoromethyl-5-(1-substituted)alkylpyridines by cyclization which avoids the use of the 4-alkoxy-1,1,1-trifluoro-3-buten-2-ones. More particularly, the present invention concerns a process for the preparation of a 2-trifluoromethyl-5-(1-substituted)alkylpyridine (I), 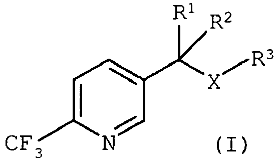 wherein
R¹ and R² independently represent H, C₁-C₄ alkyl, or either of R¹ or R² taken together with R³ represent a 4- to 6-membered saturated ring, or R¹ taken together with R² represents a 3- to 6-membered saturated ring optionally substituted with an O or a N atom;
R³ represents C₁-C₄ alkyl or R³ taken together with either of R¹ or R² represent a 4- to 6-membered saturated ring; and
X represents CH₂, O or S;
which comprises
i) contacting an alkyl vinyl ether of the formula 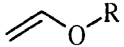 in which R represents a C₁-C₄ alkyl
   with trifluoroacetyl chloride to provide a 4-chloro-4-alkoxy-1,1,1-trifluoro-2-butanone of the formula (II): 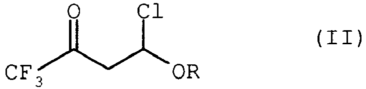 in which R is as previously defined;
ii) condensing the 4-chloro-4-alkoxy-1,1,1-trifluoro-2-butanone (II) with an enamine (III) 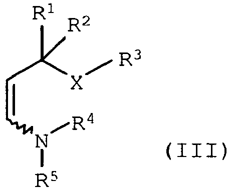 wherein
   R¹, R², R³ and X are as previously defined; and
   R⁴ and R⁵ independently represent hydrogen, C₁-C₈ alkyl, C₂-C₈ alkenyl, C₁-C₈ arylalkyl, C₁-C₈ haloalkyl, C₁-C₈ alkoxyalkyl, C₁-C₈ alkylaminoalkyl, aryl or heteroaryl or R⁴ and R⁵ taken together with N represent a 5- or 6-membered saturated or unsaturated ring;
   in the presence of a tertiary amine base to provide an intermediate of the formula (IV) 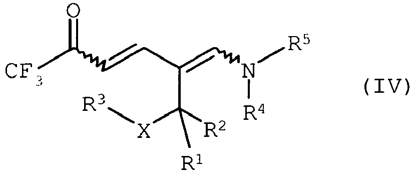 wherein
      R¹, R², R³, R⁴, R⁵ and X are as previously defined; and
iii) cyclizing the intermediate of the formula (IV) in the presence of ammonia or a reagent capable of generating ammonia.

In the preferred embodiments of the present invention, R¹ and R² independently represent H or methyl, R³ represents methyl and X represents S.

### DETAILED DESCRIPTION OF THE INVENTION

Unless specifically limited otherwise, the term "alkyl" (including derivative terms such as "haloalkyl", "alkoxyalkyl", "alkylaminoalkyl" and "arylalkyl"), as used herein, include straight chain, branched chain, and cyclic groups. Thus, typical alkyl groups are methyl, ethyl, 1-methylethyl, propyl, 1,1-dimethylethyl, and cyclopropyl. The term "alkenyl", as used herein, includes straight chain, branched chain, and cyclic groups and is intended to include one or more unsaturated bonds. The term "halogen" includes fluorine, chlorine, bromine and iodine. The term "haloalkyl" includes alkyl groups substituted with from one to the maximum possible number of halogen atoms. The term "aryl", as well as derivative terms such as "arylalkyl", refers to a phenyl or naphthyl group. The term "heteroaryl" refers to a 5-or 6-membered aromatic ring containing one or more heteroatoms, viz., N, O or S; these heteroaromatic rings may be fused to other aromatic systems.

In the present invention, a 2-trifluoromethyl-5-(1-substituted)alkylpyridine (I), 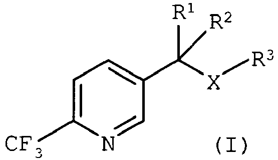 wherein
R¹ and R² independently represent H, C₁-C₄ alkyl, or either of R¹ or R² taken together with R³ represent a 4- to 6-membered saturated ring, or R¹ taken together with R² represents a 3- to 6-membered saturated ring optionally substituted with an O or a N atom;
R³ represents C₁-C₄ alkyl or R³ taken together with either of R¹ or R² represent a 4- to 6-membered saturated ring; and
X represents CH₂, O or S;
is prepared by contacting an alkyl vinyl ether of the formula 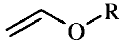 in which R represents a C₁-C₄ alkyl
with trifluoroacetyl chloride to provide a 4-chloro-4-alkoxy-1,1,1-trifluoro-2-butanone of the formula (II): 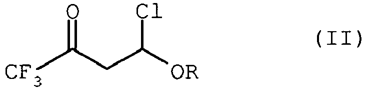 in which R is as previously defined; by condensing the 4-chloro-4-alkoxy-1,1,1-trifluoro-2-butanone (II) with an enamine (III) 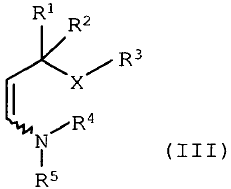 wherein
R¹, R², R³ and X are as previously defined; and
R⁴ and R⁵ independently represent hydrogen, C₁-C₈ alkyl, C₂-C₈ alkenyl, C₁-C₈ arylalkyl, C₁-C₈ haloalkyl, C₁-C₈ alkoxyalkyl, C₁-C₈ alkylaminoalkyl, aryl or heteroaryl or R⁴ and R⁵ taken together with N represent a 5- or 6-membered saturated or unsaturated ring in the presence of a tertiary amine base to provide an intermediate of the formula (IV) 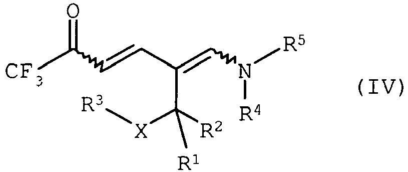 wherein
   R¹, R², R³, R⁴, R⁵and X are as previously defined; and by cyclizing the intermediate of the formula (IV) in the presence of ammonia or a reagent capable of generating ammonia.

In the first step of the present invention, 4-chloro-4-alkoxy-1,1,1-trifluoro-2-butanones of the formula (II): 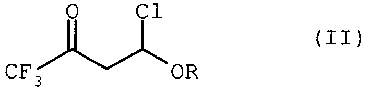 in which R represents a C₁-C₄ alkyl, are prepared by reacting an alkyl vinyl ether of the formula 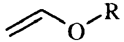 in which R represents a C₁-C₄ alkyl, with trifluoroacetyl chloride.

Approximately equimolar quantities of alkyl vinyl ether and trifluoroacetyl chloride are generally used in the process, although excesses of one or the other may be employed. In practice, a 10-50 percent stoichiometric excess of alkyl vinyl ether is preferred.

The reaction is conducted either in the absence of a solvent, e.g., with excess alkyl vinyl ether, or in the presence of an anhydrous organic solvent. Preferred solvents are hydrocarbon solvents, most preferably aromatic hydrocarbons such as toluene.

The reaction is conducted at a temperature from about -10 °C to about 35 °C. Temperatures from about 0 °C to about 20 °C are usually preferred.

In a typical reaction, the trifluoroacetyl chloride is bubbled below the surface of the alkyl vinyl ether, either neat or in the presence of a hydrocarbon solvent, between 0-5 °C. The reaction is allowed to warm with stirring for about 1 hour, keeping the temperature no higher than room temperature. The crude reaction mixture containing the 4-chloro-4-alkoxy-1,1,1-trifluoro-2-butanone is usually used as is without further isolation or purification of the reaction mixture.

In the second step of the present invention, the 4-chloro-4-alkoxy-1,1,1-trifluoro-2-butanone (II) is reacted with an enamine (III) in the presence of a tertiary amine base. 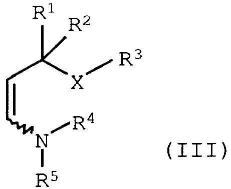 wherein
R¹, R², R³, R⁴, R⁵ and X are as previously defined to provide an intermediate of the formula (IV)

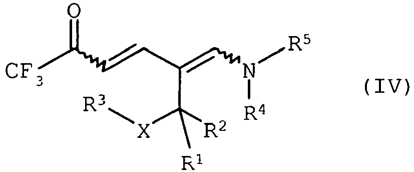

Enamines (III) can be conveniently prepared from the addition of a suitably substituted amine to an appropriately substituted aldehyde in the presence of a water adsorbing material, with or without a suitable solvent. Typically, the appropriately substituted aldehyde, for example 3-alkylthiopropionaldehyde, is reacted with an anhydrous disubstituted amine, for example pyrrolidine, at about -20 °C to about 20 °C in the presence of a desiccant such as anhydrous potassium carbonate, and the product is isolated by routine procedures and usually used without further purification.

Approximately equimolar quantities of the 4-chloro-4-alkoxy-1,1,1-trifluoro-2-butanone (II) and the enamine (III) are required in the condensation process; at least one equivalent of tertiary amine base is required with between about 1 and about 2 equivalents being preferred.

The condensation is conducted at a temperature from about -20 °C to about 35 °C. Temperatures from about -5 °C to about 20 °C are usually preferred.

This condensation is preferably conducted in a non-polar or polar aprotic solvents. Preferred non-polar solvents include hydrocarbon solvents and aromatic hydrocarbons. Polar aprotic solvents are also a good choice for this chemistry. Either acetonitrile or toluene are the most preferred solvents.

It is preferred that the 4-chloro-4-alkoxy-1,1,1-trifluoro-2-butanone (II) should be added to a preformed mixture of enamine (III) and tertiary amine base.

In a typical condensation reaction, the enamine (III) and at least a stoichiometric amount of a tertiary amine base are dissolved in the desired solvent at about -5°C to about 20°C and 4-chloro-4-alkoxy-1,1,1-trifluoro-2-butanone (II) is continuously added via addition funnel to this solution. The mixture is agitated until the 4-chloro-4-alkoxy-1,1,1-trifluoro-2-butanone (II) and enamine (III) are consumed. Intermediate (IV) is usually used as is without further isolation or purification.

In the final step of the process, the intermediate of the formula (IV) 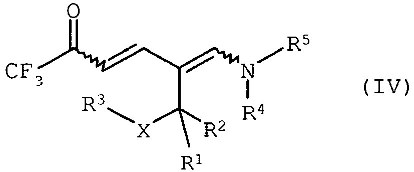 is cyclized in the presence of ammonia or a reagent capable of generating ammonia to provide the desired 2-trifluoromethyl-5-(1-substituted) alkylpyridine (I).

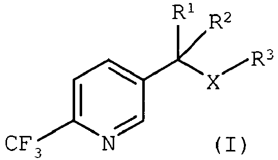

Typical reagents capable of generating ammonia include, for example, 1) an ammonium salt of an acid, preferably an organic acid, 2) formamide, or 3) formamide with an acid or acid salt. The ammonium salt of any aliphatic or aromatic organic acid can be used, but for convenience of processing, the ammonium salts of C₁-C₄ alkanoic acids are preferred. Ammonium formate and ammonium acetate are most preferred.

Approximately equimolar quantities of the intermediate (IV) and ammonia or reagents capable of generating ammonia are required in the cyclization process, although 2-4 fold excesses of the ammonia or the ammonia precursor are often preferred.

This cyclization is preferably conducted in the same solvent as the condensation.

The reaction is conducted at a temperature from about ambient temperature to about 150 °C. Temperatures from about 75 °C to about 125 °C are usually preferred.

The product is isolated by conventional techniques such as silica gel chromatography or fractional distillation.

In a typical cyclization reaction, the ammonium salt of an organic acid is added to the intermediate (IV) directly from the condensation reaction, and the mixture is heated until the reaction is complete. After dissolving in a non-water miscible solvent and washing with water and, optionally, brine, the 2-trifluoromethyl-5-(1-substituted)alkylpyridine (I) can be isolated by vacuum distillation.

The following examples are presented to illustrate the invention.

### EXAMPLES

### Example 1: Preparation of 5-(1-methylthio)ethyl-2-(trifluoromethyl)pyridine

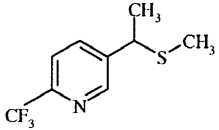

### Step 1. Preparation of 1-(3-methylthiobut-1-enyl)pyrrolidine

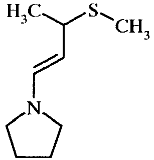

To a dry 5000 milliliter (mL) round bottom flask equipped with mechanical stirrer, nitrogen inlet, addition funnel, and thermometer, was charged 591 g (4.27 moles) of dry granular potassium carbonate and 1428 mL (17.1 moles) of anhydrous pyrrolidine. The mixture was stirred under a atmosphere of nitrogen, and cooled to 4 °C with an ice bath, after which 1050 mL (8.9 moles) of 3-methyl-thiobutyraldehyde was added at a rate that maintains the temperature below 10 °C. Upon the completion of the addition, the cooling bath was removed and the reaction was allowed to reach room temperature. The reaction contents were then filtered through a sintered glass filter funnel to remove the solids, and the solids were washed with 200 mL of anhydrous ethyl ether. The filtrate was concentrated under vacuum on a rotary evaporator until all of the pyrrolidine was removed to afford 1,519 g of 1-(3-methylthiobut-1-enyl)pyrrolidine as a red liquid. ¹H NMR CDCl₃ δ 1.36 (d, 3H ), 1.85 (m, 4H), 2.02 (s, 3H), 3.02 (m, 4H), 3.26 (q, 1H), 3.98 (dd, 1H), 6.25 (d, 1H).

### Step 2. Preparation of 5-(1-methylthio)ethyl-2-(trifluoromethyl)pyridine

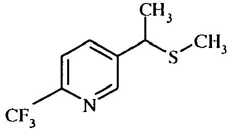

To a 100-mL three neck round bottom flask fitted with a dry-ice/acetone condenser, addition funnel, and thermowell with digital temperature monitoring was charged 8.58 g (49.0 mmol) of 1-(3-methylthiobul-1-enyl)pyrrolidine and then 40 mL of toluene. To this mixture was added 4.96 g (49.0 mmol) of triethylamine in one portion. This mixture was chilled in an ice-water bath and then 9.78 g (49 mmol) of neat 4-chloro-4-ethoxy-1,1,1-trifluorobutan-2-one was continuously added dropwise via addition funnel over a 15 min period. The internal reaction temperature rose from 4 °C to 14 °C during 4-chloro-4-ethoxy-1,1,1-trifluorobutan-2-one addition. The ice-water bath was removed, and the solution was stirred at ambient temperature for an additional 17 h. The reaction mixture was suction filtered and the filtrate collected into a 250-mL three neck round bottom flask equipped with a water-cooled condenser, thermowell, magnetic stir bar, and a bleach scrubber. To this room temperature reaction mixture was added 5.66 g (74 mmol) of ammonium acetate in one portion. The reaction mixture was then heated until the internal reaction mixture reached ~93 °C (at which time a gentle reflux was noticed inside the reaction vessel). The reaction mixture was stirred for 20 min at which time normal phase LC analysis indicated that intermediate diene of formula (IV) was still present in the reaction mixture. The reaction mixture was stirred an additional 20 min and then normal phase LC analysis indicated that the starting material was consumed. To this mixture was added 40 mL of water and the aqueous layer was separated and extracted with 15 mL of fresh toluene. The combined organic layers were concentrated on a rotovap. The residue was bulb-to-bulb distilled using a Kugelrohr apparatus (bp -80 °C at 1.5 mm Hg) to give 4.56 g (~43% yield, no purity determined) of 5-(1-methylthio)ethyl-2-(trifluoromethyl)pyridine as a liquid. ¹H NMR (300 MHz, CDCl₃) δ 1.62 (d, *J* = 8 Hz, 3H), 1.94 (s, 3H), 3.94 (q, *J* = 8 Hz, 1H), 7.67 (d, *J* = 8 Hz, 1H), 7.90 (dd, *J* = 8, 2 Hz, 1H), 8.66 (d, *J* = 2 Hz, 1H).

### Example 2: Alternate preparation of 5-(1-methylthio)ethyl-2-(trifluoromethyl)pyridine

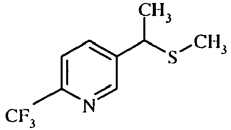

To a 500 mL jacketed reactor equipped with a circulation bath (containing syltherm 800), mechanical stirring, dry-ice/acetone condenser, and a thermowell with digital monitoring was charged with 240 mL of toluene followed by 43.3 g (0.6 mol) of ethyl vinyl ether (EVE) in one portion. The reaction mixture was cooled to 0 °C and then a ¼" Teflon line was placed sub-surface into the reaction mixture. Trifluoroacetyl chloride (TFAC) was bubbled through this Teflon line over a 1 h period until 95.3 g (0.72 mol) of trifluoroacetyl chloride reagent had been added. The internal reaction temperature rose from 2 °C to 6 °C over the TFAC addition. The circulation bath temperature was then set at 20 °C and the reaction mixture was allowed to warm up to the circulation bath set point and stirred for an additional 20 min. At this time, GC analysis indicated that EVE starting material was present in about 4% (relative GC area) and 4-chloro-4-ethoxy-1,1,1-trifluorobutan-2-one was present in about 87 % (relative GC area and subtracting the toluene peak area). This 4-chloro-4-ethoxy-1,1,1-trifluorobutan-2-one/toluene solution was collected into a glass bottle with polycap and stored for use in the condensation reaction without further purification. To a one liter three-neck round bottom flask equipped with mechanical stirring, thermowell with digital monitoring, and a reflux condenser was charged in sequence with 79.1 g (0.57 mol) of anhydrous potassium carbonate, 400 mL of toluene, and then 40.5 g (0.57 mol) pyrrolidine. The slurry was cooled using an ice-water bath and then 61.5 g (0.52 mol) of 3-methylthiobutanal was continuously added via addition funnel over a 48 min period. The internal reaction temperature was maintained below -7 °C by adjusting the addition rate of aldehyde. The ice-bath was removed and the slurry was allowed to warm to ambient temperature and stirred overnight. At this point, ¹H NMR analysis of the reaction mixture indicated complete formation of 1-(3-methylthiobut-1-enyl)pyrrolidine. The reaction mixture was suction filtered and the filter cake was rinsed with 20 mL of fresh toluene. The filtrate was partially concentrated on a rotovap until about 375 mL of distillate was collected. The resulting 1-(3-methylthiobul-1-enyl)pyrrolidine /toluene solution was taken into the condensation reaction without further purification. To a two liter three-neck round bottom flask equipped with mechanical stirring, a thermowell with digital temperature monitoring, and a reflux condenser was charged 1-(3-methylthiobut-1-enyl)pyrrolidine /toluene solution (from example 2) followed by an additional 200 mL of fresh toluene. To this mixture was added 90.9 g (0.9 mol) of triethylamine in one portion and then the reaction mixture was cooled in an ice-water bath. To this mixture was continuously added the 4-chloro-4-ethoxy-1,1,1-trifluorobutan-2-one/toluene solution (from example 2) via addition funnel over a 3 h period. The internal reaction temperature rose from 3 °C to 7 °C during the 4-chloro-4-ethoxy-1,1,1-trifluorobutan-2-one addition. The reaction mixture was stirred an additional 25 min with ice-water bath cooling and then the ice-water bath was removed. The reaction mixture was allowed to warm to ambient temperature and stirred overnight. At this point, normal phase LC was taken to determine the presence of diene intermediate of formula (IV). To this ambient temperature reaction slurry was added 55.5. g (0.72 mol) of ammonium acetate in one portion. The reaction mixture was heated up to -93 °C at which time a gentle reflux was noticed in the reaction vessel. After stirring the heated reaction mixture for 33 min, normal phase LC analysis indicated the disappearance of diene intermediate of formula (IV). To this reaction mixture was added 400 mL of cold tap water and the two-phase system was allowed to stir an additional 25 min. The reaction mixture was transferred to a two liter separatory funnel. The bottom aqueous layer (∼600 mL) was separated and discarded into a waste container for disposal. The top organic layer (∼607 g) was taken onto the final isolation/purification stage. GC assay analysis (using dibutyl phthalate as an internal standard) of the organic layer indicated a 66% "in-pot" yield of 5-(1-methylthio)ethyl-2-(trifluoromethyl)pyridine. This organic layer was concentrated on a rotovap to remove most of the toluene, and then the remaining residue was transferred to a 250 mL three-neck round bottom flask equipped with a thermowell using digital temperature monitoring and a one piece micro distillation head apparatus. A vacuum was applied and the three fractions were collected:
Fraction #1 (bp 59-70 °C at 0.5 mm Hg) collected 1.12 g of liquid.
Fraction #2 (bp 68-80 °C at ~0.1 mm Hg) collected 65.64 g of yellow liquid.
Fraction #3 (bp 58-62 °C at 0.01 mm Hg) collected 2.76 g of liquid.

Fraction #2 isolated 5-(1-methylthio)ethyl-2-(trifluoromethyl)pyridine in about 55% yield (based on starting 3-methylthiobutunal) with a purity of 97% by GC assay analysis (using dibutyl phthalate as an internal standard).

### Example 3: Alternate preparation of 5-(1-methylthio)ethyl-2-(trifluoromethyl)pyridine

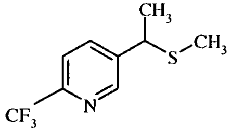

To a 2-liter three-neck round bottom flask equipped with mechanical stirring, a nitrogen pad, and a temperature probe was charged with 152.0 g (1.10 mol) of solid potassium carbonate followed by ~1 liter of toluene. To this mixture was added 71.1 g (1.00 mol) of pyrrolidine. The reaction mixture was cooled in an ice-water bath and then 118.20 g (1.00 mol) of 3-methylthiobutanal was continuously added via addition funnel over a 1 h 16 min period. Addition rate of aldehyde was adjusted so the internal reaction temperature was maintained below ~10 °C. The ice-water bath was removed and the reaction mixture was allowed to warm to ambient temperature and stirred an additional 3 h 30 min. At this time, GC analysis indicated that starting 3-methylthiobutanal was present in about -0.4% (relative area). The reaction mixture was suction filtered and the filter cake was rinsed with 250 mL of fresh toluene. The filtrate was collected and diluted with an additional 50 mL of fresh toluene. This 1-(3-methylthiobut-l-enyl)pyrrolidine /toluene solution was taken into the condensation reaction without further purification. To a 500 mL jacketed reactor equipped with a circulation bath (containing syltherm 800), mechanical stirring, and a thermowell with digital monitoring was charged with 95.2 g (1.32 mol) of ethyl vinyl ether (EVE) followed by 50 mL of toluene. The reaction mixture was cooled in an ice-water bath and 147.7 g (1.11 mol) of trifluoroacetyl chloride (TFAC) was added using a sub-surface addition line over a 2 h period. The reaction temperature was maintained below -10 °C by controlling the addition rate of TFAC gas. The reaction mixture was stirred an additional 2 h 10 min with ice-water bath cooling. At this time, GC analysis indicated that EVE starting material was present in about 4.3% (relative GC area) and 4-chloro-4-ethoxy-1,1,1-trifluorobutan-2-one was present in about 80 % (relative GC area and subtracting the toluene peak area). This 4-chloro-4-ethoxy-1,1,1-trifluorohutan-2-one/toluene solution was taken into the condensation reaction without further purification. To a 3-liter three-neck round bottom flask equipped with mechanical stirring and a temperature probe was charged with the 1-(3-methylthiobut-1-enyl)pyrrolidine /toluene solution (example 3). To this mixture was added 172.0 g (1.7 mol) of triethylamine in one portion and this mixture was cooled in an ice-water bath. To this cooled mixture was continuously added via addition funnel the 4-chloro-4-ethoxy-1,1,1-trifluorobutan-2-one/toluene solution (example 3) over a 2 h period. The internal reaction temperature rose from 4 °C to 8 °C during the 4-chloro-4-ethoxy-1,1,1-tfifluorobutan-2-one addition. After stirring for 10 min with ice-water bath cooling, the ice-water bath was removed and the reaction mixture was allowed to warm to ambient temperature and stirred overnight (∼15 h). At this point, normal phase LC was taken to determine the presence of diene intermediate of formula (IV). To this mixture was added 100 g (1.30 mol) of ammonium acetate in one portion. The reaction mixture was heated up to -90 °C at which time a gentle reflux was noticed in the reaction vessel. After stirring the heated reaction mixture for 2 h and 22 min, normal phase LC analysis indicated the disappearance of diene intermediate of formula (IV). The reaction mixture was cooled to ambient temperature, 500 mL of cold tap water was added, and then the mixture was allowed to stir for 2 h 20 min. The bottom aqueous layer (∼780 mL) was discarded and the top organic layer was concentrated on a rotovap to give 275 g of a dark brown oil. GC assay analysis (using dibutyl phthalate as an internal standard) of the oil indicated a 69% "in-pot" yield of 5-(1-methylthio)ethyl-2-(trifluoromethyl)pyridine. The crude residue was transferred to a 500 mL round bottom flask equipped with a thermowell using digital temperature monitoring and a one piece micro distillation head. A vacuum was applied and one fraction was collected: (bp 101-125 °C at 12 mm Hg) collected 132 g of liquid. 5-(1-Methylthio)ethyl-2-(trifluoromethyl)pyridine was isolated in 56% yield (based on starting 3-methylthiobutunal) with a purity of 94% by GC assay analysis (using dibutyl phthalate as an internal standard). ¹H NMR of the isolated product was similar to that reported for example 1, step 2.

### Example 4: Preparation of 5-ethyl-2-trifluromethylpyridine

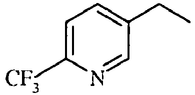

To a 500 mL jacketed reactor equipped with a circulation bath, mechanical stirring, dry-ice/acetone condenser, and a thermowell with digital monitoring was charged 100 mL of toluene followed by 45.2 g (0.63 mol) of ethyl vinyl ether in one portion. The reaction mixture was cooled to 2 °C and then a Teflon line was placed sub-surface into the reaction mixture. Trifluoroacetyl chloride (TFAC) was then bubbled through this Teflon line over a 1 h period until 99.9 g (0.75 mol) of TFAC reagent had been added. The internal reaction temperature rose from 2 °C to 6 °C over the TFAC addition. The circulation bath temperature was then set to 20 °C and the reaction mixture was allowed to warm up to the circulation bath set point and stirred for an additional 1 h. At this time, GC analysis indicated that 4-chloro-4-ethoxy-1,1,1-trifluorobutan-2-one was present in about 89 % (relative GC area). This 4-chloro-4-ethoxy-1,1,1-trifluorobutan-2-one/toluene solution was collected into a glass bottle with polycap and used in the condensation reaction without further purification. A one-liter three-neck round bottom flask equipped with mechanical stirring, thermowell with digital monitoring, and a reflux condenser was charged in sequence with 78.8 g (0.57 mol) of anhydrous potassium carbonate, 400 mL of toluene, and then 40.5 g (0.57 mol) pyrrolidine. The slurry was cooled in an ice-water bath and 37.5 g (0.52 mol) of butanal was continuously added via addition funnel over a 1 h period. The internal reaction temperature was maintained below -7 °C by adjusting the addition rate of aldehyde. After stirring the reaction mixture for 46 min with ice-water bath cooling, the ice-bath was removed and the slurry was allowed to warm to ambient temperature and stirred overnight. At this point, GC analysis of the reaction mixture indicated complete formation of 1-(but-1-enyl)pyrrolidine. The reaction mixture was suction filtered and the filtrate was collected into a one-liter three-neck round bottom flask equipped with a magnetic stir bar, thermowell with digital temperature monitoring, and a one piece distillation head. A partial distillation was performed to remove about 275 mL of toluene solvent (bp 60-62 °C at 150 mm Hg). The 1-(but-1-enyl)pyrrolidine /toluene solution was taken into the condensation reaction without further purification. To a one-liter three-neck round bottom flask equipped with mechanical stirring, a thermowell with digital temperature monitoring, and a reflux condenser was charged with the 1-(but-1-enyl)pyrrolidine /toluene solution (example 4). To this mixture was added 93.9 g (0.93 mol) of triethylamine in one portion and the reaction mixture was cooled in an ice-water bath. To this mixture was continuously added via addition funnel the 4-chloro-4-ethoxy-1,1,1-trifluorobutan-2-one/toluene solution (example 4) over a -3 h period. The internal reaction temperature rose from 2 °C to 10 °C during the 4-chloro-4-ethoxy-1,1,1-trifluorobutan-2-one addition. The reaction mixture was stirred an additional 5 min with ice-water bath cooling and then the ice-water bath was removed. The reaction mixture was allowed to warm to ambient temperature and stirred overnight. At this point, GC analysis indicated the presence of both unreacted 1-(but-1-enyl)pyrrolidine (and diene intermediate of formula (IV). The reaction mixture was then heated to 50 °C and stirred for about 1 h. GC analysis of the reaction mixture indicated the disappearance of 1-(but-1-enyl)pyrrolidine. After cooling the reaction mixture to approximately room temperature, 60 g (0.78 mol) of ammonium acetate was added in one portion. The reaction mixture was heated up to -90 °C at which time a gentle reflux was noticed in the reaction vessel. After stirring the heated reaction mixture for 30 min, normal phase LC analysis indicated that diene intermediate of formula (IV) was still present in the reaction mixture. After stirring the reaction mixture for an additional 45 min, normal phase LC indicated no change in the reaction mixture composition. An additional 20 g (0.26 mol) of ammonium acetate was added to the reaction mixture in one portion. The reaction mixture was stirred an additional 30 min and normal phase LC analysis indicated no change from those previously taken. To this reaction mixture was added 200 mL of cold tap water and the reaction mixture was transferred to a 2-liter separatory funnel. The bottom aqueous layer was separated and discarded into a waste container for disposal. The top organic layer (~336 g) was taken onto the final isolation/purification stage. GC assay analysis of the organic layer indicated a 71% "in-pot" yield of crude 5-ethyl-2-trifluromethylpyridine. This organic layer was transferred to a 500 mL three-neck round bottom flask equipped with a magnetic stir bar, thermowell with digital temperature monitoring, and a one piece distillation head. A vacuum was applied and the three fractions were collected:
Fraction #1 (bp 45-67 °C at 156 mm Hg) collected 192 g of liquid.
Fraction #2 (bp 40-47 °C at 54 mm Hg) collected 41 g of yellow liquid.
Fraction #3 (bp 78-81 °C at 15 mm Hg) collected 59 g of liquid.

Fraction #3 isolated 5-ethyl-2-trilluoromethylpyridine in about 62% yield (based on starting butanal) with a purity of 96% by GC assay analysis (using dipropyl phthalate as an internal standard). ¹H NMR (300 MHz, CDCl₃) δ 1.27 (t, *J* = 7.5 Hz, 3H), 2.74 (q, *J* = 7.5 Hz, 2H), 7.60 (d, *J* = 8.0 Hz, 1H), 7.68 (dq, *J* = 8.0, 0.6 Hz, 1H), 8.57 (s, 1H). ¹³C NMR (75.5 MHz, CDCl₃) δ 14.9, 25.9, 120.1 (q, *J* = 2.7 Hz), 121.7 (q, *J* = 274 Hz), 136.4, 142.6, 145.8 (q, *J* = 34 Hz), 149.8. GC/EIMS (relative peak intensity) *m*/*z* 175 (90), 160 (100), 106 (35).

## Claims

1. A process for the preparation of a 2-trifluoromethyl-5-(1- substituted)alkylpyridine (I), 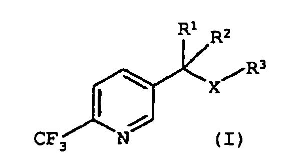 wherein
R¹ and R² independently represent H, C₁-C₄ alkyl, or either of R¹ or R² taken together with R³ represent a 4- to 6-membered saturated ring, or R¹ taken together with R² represents a 3- to 6-membered saturated ring optionally substituted with an O or a N atom;
R³ represents C₁-C₄ alkyl or R³ taken together with either of R¹ or R² represent a 4- to 6-membered saturated ring; and
X represents CH₂, O or S;
which comprises
i) contacting an alkyl vinyl ether of the formula 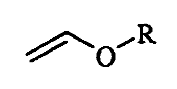 in which R represents a C₁-C₄ alkyl
with trifluoroacetyl chloride to provide a 4-chloro-4-alkoxy- 1,1,1 -trifluoro-2-butanone of the formula (II): 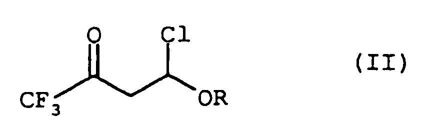 in which R is as previously defined;
ii) condensing the 4-chloro-4-alkoxy-I,I,I-trifluoro-2-butanone (II) with an enamine (III) 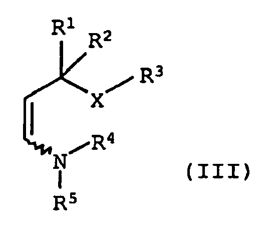 wherein
R¹, R², R³ and X are as previously defined; and
R⁴ and R⁵ independently represent hydrogen, C₁-C₈ alkyl, C₂-C₈ alkenyl, C₁-C₈ arylalkyl, C₁-C₈ haloalkyl, C₁-C₈ alkoxyalkyl, C₁-C₈ alkylaminoalkyl, aryl or heteroaryl or R⁴ and R taken together with N represent a 5- or 6-membered saturated or unsaturated ring;
in the presence of a tertiary amine base to provide an intermediate of the formula (IV) 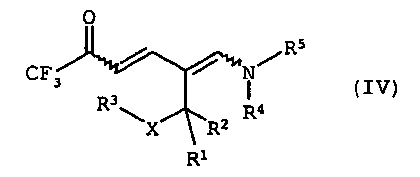 wherein
R¹, R², R³, R⁴, R⁵ and X are as previously defined;
wherein alkyl includes in each case straight chain, branched chain, and cyclic groups and
wherein alkenyl includes in each case straight chain, branched chain, and cyclic groups; and
iii) cyclizing the intermediate of the formula (IV) in the presence of ammonia or a reagent capable of generating ammonia.

2. The process of Claim 1 in which R¹ and R² independently represent H or methyl, R³ represents methyl and X represents S.

3. The process of claim 1 in which the reagent capable of generating ammonia is an ammonium salt of an organic acid.

## Patentansprüche

1. Verfahren zur Herstellung von einem 2-Trifluormethyl-5-(1- substituiertem)alkylpyridin (I), 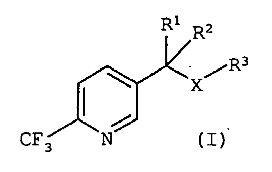 wobei
R¹ und R² unabhängig voneinander H, C₁-C₄-Alkyl darstellen, oder entweder R¹ oder R² zusammen mit R³ einen 4- bis 6-gliedrigen gesättigten Ring darstellen, oder R¹ zusammen mit R² einen 3- bis 6-gliedrigen gesättigten Ring darstellt, ggf. mit einem O- oder einem N-Atom substituiert;
R³ C₁-C₄-Alkyl darstellt oder R³ zusammen mit entweder R¹ oder R² einen 4- bis 6-gliedrigen gesättigten Ring darstellt; und
X CH₂, O oder S darstellt;
welcher umfasst
i) Inkontaktbringen eines Alkylvinylethers der Formel 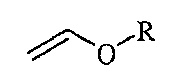 in welcher R ein C₁-C₄-Alkyl darstellt
mit Trifluoracetylchlorid zur Bereitstellung eines 4-Chlor-4-alkoxy-1,1,1-trifluor-2-butanon von der Formel (II): 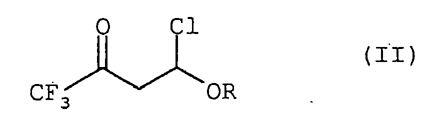 in welcher R wie zuvor definiert ist;
ii) Kondensieren des 4-Chlor-4-alkoxy-I,I,I-trifluor-2-butanon (II) mit einem Enamin (III) 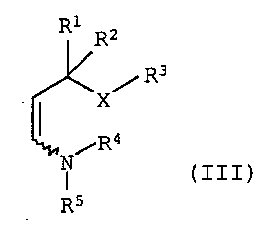 wobei
R¹, R², R³ und X wie vorher definiert sind; und
R⁴ und R⁵ unabhängig Wasserstoff, C₁-C₈-Alkyl, C₂-C₈-Alkenyl, C₁-C₈-Arylalkyl, C₁-C₈-Halogenalkyl, C₁-C₈-Alkoxyalkyl, C₁-C₈-Alkylaminoalkyl, Aryl oder Heteroaryl darstellen oder R⁴ und R⁵ zusammen mit N einen 5- oder 6-gliedrigen gesättigten oder ungesättigten Ring darstellen;
in der Gegenwart von einer tertiären Aminbase zur Bereitstellung eines Zwischenprodukts von der Formel (IV) 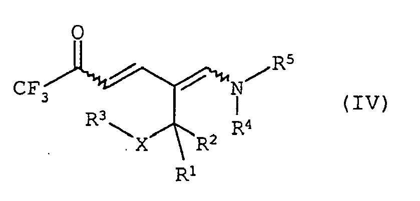 wobei
R¹, R², R³, R⁴, R⁵ und X wie vorher definiert sind;
wobei Alkyl in jedem Fall gerade Ketten, verzweigte Ketten und zyklische Gruppen einschließt
und
wobei Alkenyl in jedem Fall gerade Ketten, verzweigte Ketten und zyklische Gruppen einschließt; und
iii) Zyklisieren des Zwischenprodukts von der Formel (IV) in der Gegenwart von Ammoniak oder einem Reagenz, welches fähig ist Ammoniak zu erzeugen.

2. Verfahren nach Anspruch 1, in welchem R¹ und R² unabhängig voneinander H oder Methyl darstellen, R³ Methyl darstellt und X S darstellt.

3. Verfahren nach Anspruch 1, in welchem das Reagenz, welches fähig ist Ammoniak zu erzeugen, ein Ammoniumsalz einer organischen Säure ist.

## Revendications

1. Procédé de préparation d'une 2-trifluorométhyl-5-(alkyle à substituant en position 1)-pyridine, de formule (I) : 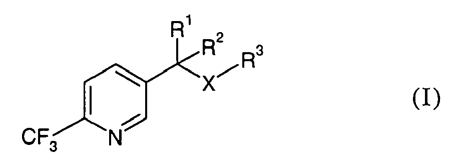 dans laquelle
- R¹ et R² représentent chacun, indépendamment, un atome d'hydrogène ou un groupe alkyle en C₁-C₄, ou l'un ou l'autre de ces symboles R¹ et R², considéré conjointement avec R³, représente un cycle saturé comportant de 4 à 6 chaînons, ou encore les symboles R¹ et R², considérés conjointement, représentent un cycle saturé comportant de 3 à 6 chaînons, dont l'un, en option, peut être remplacé par un atome d'oxygène ou d'azote ;
- R³ représente un groupe alkyle en C₁-C₄, ou R³, considéré conjointement avec l'un ou l'autre des symboles R¹ et R², représente un cycle saturé comportant de 4 à 6 chaînons ;
- et X représente un chaînon méthylène CH₂, ou un atome d'oxygène ou de soufre ;
lequel procédé comporte les étapes suivantes :
i) mettre un alkyl-vinyl-éther, de formule 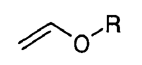 dans laquelle R représente un groupe alkyle en C₁-C₄,
en contact avec du chlorure de trifluoroacétyle, de manière à ce qu'il se forme une 4-chloro-4-alcoxy-1,1,1-trifluoro-butan-2-one, de formule (II) : 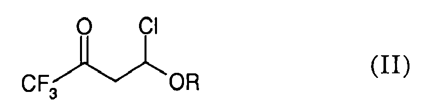 dans laquelle R a la signification indiquée ci-dessus ;
ii) condenser cette 4-chloro-4-alcoxy-1,1,1-trifluoro-butan-2-one (II) avec une énamine, de formule (III) : 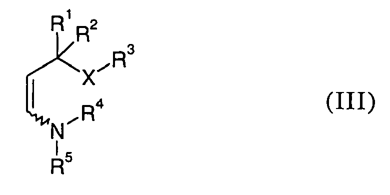 dans laquelle
- R¹, R², R³ et X ont les significations indiquées plus haut,
- et R⁴ et R⁵ représentent chacun, indépendamment, un atome d'hydrogène ou un groupe alkyle en C₁-C₈, alcényle en C₂-C₈, aryl-alkyle en C₁-C₈, halogénoalkyle en C₁-C₈, alcoxy-alkyle en C₁-C₈, alkyl-amino-alkyle en C₁-C₈, aryle ou hétéroaryle,
- ou R⁴ et R⁵, considérés conjointement avec N, représentent un cycle saturé ou insaturé, comportant 5 ou 6 chaînons,
en présence d'une base de type amine tertiaire, de manière à ce qu'il se forme un produit intermédiaire, de formule (IV) : 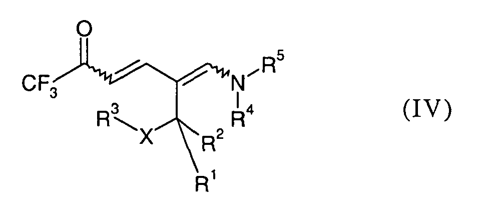 dans laquelle R¹, R², R³, R⁴, R⁵ et X ont les significations indiquées plus haut,
étant entendu que le terme "alkyle" inclut, en chaque occurrence, les groupes à chaîne linéaire, les groupes à chaîne ramifiée et les groupes cycliques, et que le terme "alcényle" inclut, en chaque occurrence, les groupes à chaîne linéaire, les groupes à chaîne ramifiée et les groupes cycliques ;
iv) et opérer la cyclisation de ce produit intermédiaire de formule (IV), en présence d'ammoniac ou d'un réactif capable de produire de l'ammoniac.

2. Procédé conforme à la revendication 1, dans lequel R¹ et R² représentent chacun, indépendamment, un atome d'hydrogène ou un groupe méthyle, R³ représente un groupe méthyle et X représente un atome de soufre.

3. Procédé conforme à la revendication 1, dans lequel le réactif capable de produire de l'ammoniac est un sel d'ammonium d'un acide organique.
